# EUROPÄISCHE PATENTSCHRIFT

(11) **EP 1 000 612 B1**
(45) Veröffentlichungstag und Bekanntmachung des Hinweises auf die Patenterteilung: **06.08.2003**
(21) Anmeldenummer: 99120947.9
(22) Anmeldetag: 02.11.1999
(51) Int. Cl.: A61K 7/48

(54) **Zubereitungen vom Emulsionstyp W/O mit erhöhtem Wassergehalt, enthaltend ferner ein oder mehrere Alkylmethiconcopolyole und/oder Alkyl-Dimethiconcopolyole sowie kationische Polymere**
Preparations of W/O type emulsions with high water content and further containing one or more alkylmethicone copolyols and/or alkyl-dimethicone copolyols as well as cationic polymers
Préparations d'émulsions du type E/H à haute teneur en eau et contenant en plus un ou plusieurs alkylméthicone copolyols et/ou alkyl-diméthicone coplyols ainsi que des polymères cationiques

(30) Priorität: 12.11.1998 DE 19852212
(43) Veröffentlichungstag der Anmeldung: 17.05.2000
(73) Patentinhaber: Beiersdorf Aktiengesellschaft, 20245 Hamburg (DE)
(72) Erfinder: Bleckmann, Andreas, 22926 Ahrensburg (DE); Kröpke, Rainer, 22849 Schenefeld (DE); Schneider, Günther, Dr., 22607 Hamburg (DE)

(56) Entgegenhaltungen:
- EP-A- 0 424 260
- WO-A-95/22311

## Beschreibung

Die vorliegende Erfindung betrifft kosmetische und dermatologische Zubereitungen, insbesondere solche vom Typ Wasser-in-ÖI, Verfahren zu ihrer Herstellung sowie ihre Verwendung für kosmetische und medizinische Zwecke.

Die menschliche Haut übt als größtes Organ des Menschen zahlreiche lebenswichtige Funktionen aus. Mit durchschittlich etwa 2 m2 Oberfläche beim Erwachsenen kommt ihr eine herausragende Rolle als Schutz- und Sinnesorgan zu. Aufgabe dieses Organs ist es, mechanische, thermische, aktinische, chemische und biologische Reize zu vermitteln und abzuwehren. Außerdem kommt ihr eine bedeutende Rolle als Regulations- und Zielorgan im menschlichen Stoffwechsel zu.

Unter kosmetischer Hautpflege ist in erster Linie zu verstehen, die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) zu stärken oder wiederherzustellen sowie ihre Homschicht bei aufgetretenen Schäden in ihrem natürlichen Regenerationsvermögen zu unterstützen.

Werden die Barriereeigenschaften der Haut gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliches Waschen verursachten Fettund Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittelund Arzneimittelgesetz).

Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Natürlich ist dem Fachmann eine Vielzahl von Möglichkeiten bekannt, stabile W/O-Zubereitungen zur kosmetischen oder dermatologischen Anwendung zu formulieren, beispielsweise in Form von Cremes und Salben, die im Bereich von Raumbis Hauttemperatur streichfähig sind, oder als Lotionen und Milche, die in diesem Temperaturbereich eher fließfähig sind.

Eine Aufgabe der vorliegenden Erfindung war es, Zubereitungen zur Verfügung zustellen, welche mit einem hohen oder sehr hohen Gehalt an wasserlöslichen und/oder wassermischbaren Substanzen formuliert werden können, ohne daß die galenische Qualität oder andere Eigenschaften der Zubereitungen beeinträchtigt wären.

Als "High Intemal Phase Emulsions" werden nach K.J.Lissant: The Geometry of High-lntemal-Phase-Ratio Emulsions; Journal of Colloid and Interface Science 22, 462-468 (1966) Emulsionen mit einer inneren Phase von > 70% definiert. Die Herstellung von stabilen, festen bis halbfesten " High Internal Phase Water-in-Oil Emulsions", insbesondere solche mit einem höheren Wassergehalt als 80% ("Very High Internal Phase Water-in-Oil Emulsions") und mit dennoch sehr guten sensorischen Eigenschaften, stellt sich als ungelöstes Problem dar. Durch den sehr hohen Wassergehalt der Emulsionen "brechen" diese auf der Haut besonders schnell - sensorisch unangenehm - in ihre Hauptbestandteile (hydrophile und lipophile Komponenten) auf. Weiterhin trennen sich auch die lipophilen Komponenten in ihre Einzelbestandteile auf, so daß die Lipide auf der Haut voneinander - sensorisch unangenehm - "abglitschen".

Die üblicherweise bei Wasser-in-Öl-Emulsionen angewandte Technik der Variation des Phasen-Volumen-Verhältnisses (d.h. Einarbeitung höherer Mengen an flüssigen Lipiden) kann, auf Grund des niedrigen Lipidanteils bei "High Internal Phase"-W/O-Emulsionen nur bedingt, bei "Very High Internal Phase"-W/O-Emulsionen) überhaupt nicht genutzt werden

Überraschend hat sich gezeigt, daß Wasser-in-Öl-Emulsionen
(a) eines Gehaltes an Wasser und gegebenenfalls wasserlöslichen Substanzen von insgesamt mindestens 80 Gew.-%, bezogen auf das Gesamtgewicht der Zubereitungen und eines Gehaltes an Lipiden, Emulgatoren und lipophilen Bestandteilen von insgesamt höchstens 20 Gew.-%.
(b) enthaltend wenigstens eine grenzflächenaktive Substanz, gewählt aus der Gruppe der Alkylmethiconcopolyole und/oder Alkyl-Dimethiconcopolyole,
(c) enthaltend eine oder mehrere Lipidkomponenten, welche die Lipidphase der Emulsion umfassen
(d) wobei das Gewichtsverhältnis von (b) zu (c) aus dem Bereich 0,10 bis 0,25 gewählt wird,
(e) ferner enthaltend mindestens ein kationisches Polymer,
den Nachteilen des Standes der Technik abhelfen.

Erstaunlicherweise sind die erfindungsgemäßen Zubereitungen in der Anwendung auf der Haut äußerst angenehm und zeichnen sich durch sehr hohe kosmetische Eleganz aus. Es ist mit den Mitteln, die dem Fachmanne ohne weiteres erfinderisches Zutun geläufig sind, nahezu beliebige Viskositäten erreichbar, so daß die vorliegende Erfindung beispielsweise als fließfähige Darreichungsform (etwa eine Lotion) oder als halbfeste bis feste Zubereitung (etwa als eine Crème) ausgestaltet werden kann.

Ein Beispiel für besonders vorteilhaft im Sinne der vorliegenden Erfindung zu verwendende grenzflächenaktiven Substanzen ist das Cetyl Dimethiconcopolyol, welches von der Gesellschaft Th.Goldschmidt AG unter der Warenbezeichnung ABIL ® EM 90 verkauft wird.

Weiterhin hat sich als ganz besonders vorteilhaft der Emulgator Laurylmethiconcopolyol herausgestellt, welcher unter der Warenbezeichnung Dow Corning® 5200 Formulation Aid von der Gesellschaft Dow Corning Ltd. erhältlich ist.

Die Gesamtmenge an den erfindungsgemäß verwendeten grenzflächenaktiven Substanzen in den fertigen kosmetischen oder dermatologischen Zubereitungen wird vorteilhaft aus dem Bereich von 0,075 - 7,5 Gew.-%, bevorzugt 0,1- 5,0 Gew.-%, insbesondere 1,0 - 3,0 Gew.-% gewählt, bezogen auf das Gesamtgewicht der Zubereitungen.

Besonders vorteilhaft enthalten die erfindungsgemäßen Zubereitungen mehr als 85 Gew.-%, insbesondere mehr als 88 Gew.-% an Wasser und gegebenenfalls wasserlöslichen Substanzen, bezogen auf das Gesamtgewicht der Zubereitungen.

Überraschend hat sich insbesondere gezeigt, daß durch den Zusatz von 0,01 bis 10% (bevorzugt 0,25 - 1,25 %) geeigneter kationischer Polymere stabile, feste bis halbfeste und/oder fließfähige "Very High Internal Phase Emulsions" hergestellt werden können, die über hervorragende sensorische Eigenschaften verfügen.

Geeignete kationische Polymere sind beispielsweise kationische Cellulosederivate (z.B. Polymer JR 400® von Amerchol), kationische Stärke, Copolymere von Diallylammoniumsalzen und Acrylamiden, quatemisierte Vinylpyrrolidon/Vinyl-imadazol-Polymere (z.B. Luviquat® von der BASF), Kondensationsprodukte von Polyglycolen und Aminen, quaternisierte Kollagenpolypeptide (z.B. Lamequat® L von Grünau-Henkel), quatemisierte Weizenpolypeptide, Polyethylenimin, kationische Silikonpolymere, Copolymere der Adipinsäure mit Dimethylaminohydroxypropyldietylentriamin, Copolymere der Acrylsäure mit Dimethyldiallylamoniumchlorid (z.B. Merquat®550 von Chemviron), Polyaminopolyamide, kationische Chitinderivate, kationischer Guar-Gum (z.B. Jaguar® CBS von Hoechst Celanese), quatemisierte Ammoniumsalz-Polymere (z.B. Mirapol® AD-1 von Miranol) sowie kationische Biopolymere wie z.B. Chitosan (mittleres Molekulargewicht von 50.000 bis 2.000.000 g/mol [bestimmt mittels Gelpermeationschromatographie] und einem Deacylierungsgrad von 10 bis 99% [bestimmt mittels 1H-NMR]).

Im Rahmen der vorliegenden Offenbarung wird als Oberbegriff für Fette, Öle, Wachse und dergleichen gelegentlich der Ausdruck "Lipide" verwendet, wie dem Fachmanne durchaus geläufig ist. Auch werden die Begriffe "Ölphase" und "Lipidphase" synonym angewandt.

Vorteilhaft können die Bestandteile der Lipidphase gewählt werden aus der Gruppe der polaren und der unpolaren Lipidkomponenten, beispielsweise gewählt aus der Gruppe Vaseline (Petrolatum), Paraffinöl und Polyolefine. Unter den Polyolefinen sind Polydecene und Hydriertes Polyisobutene die bevorzugten Substanzen.

Die Ölphase kann im Sinne der vorliegenden Erfindung ferner vorteilhaft Substanzen enthalten, gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, wie z.B. Jojobaöl.

Femer kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkemöl und dergleichen mehr.

Erfindungsgemäß vorteilhaft in der Ölphase einzusetzende Fett- und/oder Wachskomponenten können aus der Gruppe der pflanzlichen Wachse, tierischen Wachse, Mineralwachse und petrochemischen Wachse gewählt werden. Erfindungsgemäß günstig sind beispielsweise Candelillawachs, Camaubawachs, Japanwachs, Espartograswachs, Korkwachs, Guarumawachs, Reiskeimölwachs, Zuckerrohrwachs, Beerenwachs, Ouricurywachs, Montanwachs, Jojobawachs, Shea Butter, Bienenwachs, Schellackwachs, Walrat, Lanolin (Wollwachs), Bürzelfett, Ceresin, Ozokerit (Erdwachs), Paraffinwachse und Mikrowachse.

Weitere vorteilhafte Fett- und/oder Wachskomponenten sind chemisch modifzierte Wachse und synthetische Wachse, wie beispielsweise die unter den Handelsbezeichnungen Syncrowax HRC (Glyceryltribehenat), Syncrowax HGLC (C16-36-Fettsäuretriglycerid) und Syncrowax AW 1C (C18-36 -Fettsäure) bei der CRODA GmbH erhältlichen sowie Montanesterwachse, Sasolwachse, hydrierte Jojobawachse, synthetische oder modifizierte Bienenwachse (z. B. Dimethicon Copolyol Bienenwachs und/oder C30-50 -Alkyl Bienenwachs), Polyalkylenwachse, Polyethylenglykolwachse, aber auch chemisch modifzierte Fette, wie z. B. hydrierte Pflanzenöle (beispielsweise hydriertes Ricinusöl und/oder hydrierte Cocosfettglyceride), Triglyceride, wie beispielsweise Trihydroxystearin, Fettsäuren, Fettsäureester und Glykolester, wie beispielsweise C20-40-Alkylstearat, C20-40-Alkylhydroxystearoylstearat und/oder Glykolmontanat. Weiter vorteilhaft sind auch bestimmte Organosiliciumverbindungen, die ähnliche physikalische Eigenschaften aufweisen wie die genannten Fett- und/oder Wachskomponenten, wie beispielsweise Stearoxytrimethylsilan.

Erfindungsgemäß können die Fett- und/oder Wachskomponenten sowohl einzeln als auch im Gemisch vorliegen.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als Lipidkomponente der Ölphase einzusetzen.

Von den Kohlenwasserstoffen sind Paraffinöl, hydrierte Polyolefine (z.B. hydriertes Polyisobuten) Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft können Cyclomethicone (wie z.B. Cyclotetrasiloxan sowie Cyclopentasiloxan) eingesetzt werden. Aber auch andere Silikonöle sind vorteilhaft im Sinne der vorliegenden Erfindung zu verwenden, beispielsweise Dimethicone (Polydimethylsiloxane unterschiedlicher Kettenlänge wie z.B. Wacker AK 10, 20, 35, 50, 100) sowie Polymethylphenylsiloxane (wie z.B. Phenyltrimethicone).

Erfindungsgemäß vorteilhaft kann die Ölphase auch Bestandteile umfassen, gewählt aus der Gruppe der klassischen Fette, also im wesentlichen Triglyceriden.

Die erfindungsgemäßen Öle werden vorzugsweise gewählt aus der Gruppe der Triglyceride folgender Struktur 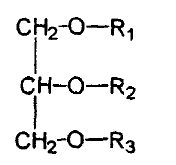 wobei R1, R2 und R3 unabhängig voneinander gewählt werden aus der Gruppe der verzweigten und unverzweigten, Alkylcarboxyl- bzw. Alkenylcarboxylgruppen mit 12 bis 24 Kohlenstoffatomen. Es ist gegebenenfalls vorteilhaft, wenn eine oder mehrere aliphatische Wasserstoffatome der Alkylcarboxyl- bzw. Alkenylcarboxylgruppen durch Hydroxylgruppen substituiert sind.

Insbesondere ist vorteilhaft, wenn R1, R2 und/oder R3 16 bis 20 Kohlenstoffatome aufweisen und aus der Gruppe der einfach bis dreifach ungesättigten Carbonsäurereste gewählt werden.

Wenn R1, R2 und/oder R3 Hydroxylgruppen tragen, ist der bevorzugte Alkenylcarboxylrest der Ricinolsäurerest.

Besonders vorteilhaft ist auch, Öle aus der Gruppe Sojaöl, Sonnenblumenöl, Weizenkeimöl und Ricinusöl zu wählen.

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, oder auch der Typen ETD (Easy-to-disperse) 2001, 2020, 2050, jeweils einzeln oder in beliebigen Kombinationen untereinander.

Ein besonderer Vorzug der vorliegenden Erfindung ist es, daß sie gestattet, hohe Konzentrationen an Polyolen, insbesondere Glycerin einzusetzen.

Besonders vorteilhafte Zubereitungen werden femer erhalten, wenn als Zusatzoder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Camosin und deren Derivate (z.B. Anserin), Carotinoide, Carotine (z.B. α-Carotin, β -Carotin, Ψ-Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µ mol/kg), femer (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin E-Acetat), Vitamin A und Derivate (Vitamin A-palmitat) sowie Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnS04) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Besonders vorteilhaft im Sinne der vorliegenden Erfindung können öllösliche Antioxidantien eingesetzt werden.

Eine erstaunliche Eigenschaft der vorliegenden Erfindung ist, daß erfindungsgemäße Zubereitungen sehr gute Vehikel für kosmetische oder dermatologische Wirkstoffe in die Haut sind, wobei bevorzugte Wirkstoffe Antioxidantien sind, welche die Haut vor oxidativer Beanspruchung schützen können. Bevorzugte Antioxidantien sind dabei Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Es ist dem Fachmann natürlich bekannt, daß kosmetische Zubereitungen zumeist nicht ohne die üblichen Hilfs- und Zusatzstoffe denkbar sind. Die erfindungsgemäßen kosmetischen und dermatologischen Zubereitungen können dementsprechend ferner kosmetische Hilfsstoffe enthalten, wie sie üblicherweise in solchen Zubereitungen verwendet werden, beispielsweise Konsistenzgeber, Stabilisatoren, Füllstoffe, Konservierungsmittel, Parfüme, Substanzen zum Verhindern des Schäumens, Farbstoffe, Pigmente, die färbende Wirkung haben, Verdickungsmittel, oberflächenaktive Substanzen, Emulgatoren, weichmachende, anfeuchtende und/oder feuchthaltende Substanzen, entzündungshemmende Substanzen, zusätzliche Wirkstoffe wie Vitamine oder Proteine, Lichtschutzmittel, Insektenrepellentien, Bakterizide, Viruzide, Wasser, Salze, antimikrobiell, proteolytisch oder keratolytisch wirksame Substanzen, Medikamente oder andere übliche Bestandteile einer kosmetischen oder dermatologischen Formulierung wie Alkohole, Polyole, Polymere, Schaumstabilisatoren, organische Lösungsmittel oder auch Elektrolyte.

Letztere können beispielsweise gewählt werden aus der Gruppe der Salze mit folgenden Anionen: Chloride, femer anorganische Oxo-Element-Anionen, von diesen insbesondere Sulfate, Carbonate, Phosphate, Borate und Aluminate. Auch auf organischen Anionen basierende Elektrolyte sind vorteilhaft, z.B. Lactate, Acetate, Benzoate, Propionate, Tartrate, Citrate, Aminosäuren, Ethylendiamintetraessigsäure und deren Salze und andere mehr. Als Kationen der Salze werden bevorzugt Ammonium,- Alkylammonium,- Alkalimetall-, Erdalkalimetall,- Magnesium-, Eisen- bzw. Zinkionen verwendet. Es bedarf an sich keiner Erwähnung, daß in Kosmetika nur physiologisch unbedenkliche Elektrolyte verwendet werden sollten. Besonders bevorzugt sind Kaliumchlorid, Kochsalz, Magnesiumsulfat, Zinksulfat und Mischungen daraus.

Mutatis mutandis gelten entsprechende Anforderungen an die Formulierung medizinischer Zubereitungen.

Die erfindungsgemäßen W/O-Emulsionen können als Grundlage für kosmetische oder dermatologische Formulierungen dienen. Diese können wie üblich zusammengesetzt sein und beispielsweise zur Behandlung und der Pflege der Haut und/oder der Haare, als Lippenpflegeprodukt, als Deoprodukt und als Schmink- bzw. Abschminkprodukt in der dekorativen Kosmetik oder als Lichtschutzpräparat dienen. Zur Anwendung werden die erfindungsgemäßen kosmetischen und dermatologischen Zubereitungen in der für Kosmetika oder Dermatika üblichen Weise auf die Haut und/oder die Haare in ausreichender Menge aufgebracht.

Entsprechend können kosmetische oder topische dermatologische Zusammensetzungen im Sinne der vorliegenden Erfindung, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Die dünnflüssigen kosmetischen oder dermatologischen Mittel gemäß der Erfindung können beispielsweise als aus Aerosolbehältem, Quetschflaschen oder durch eine Pumpvorrichtung versprühbare Präparate vorliegen oder in Form einer mittels Rollon-Vorrichtungen auftragbaren flüssigen Zusammensetzung, jedoch auch in Form einer aus normalen Flaschen und Behältern auftragbaren Emulsion.

Als Treibmittel für aus Aerosolbehältern versprühbare kosmetische oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Günstig sind auch solche kosmetischen und dermatologischen Zubereitungen, die in der Form eines Sonnenschutzmittels vorliegen. Vorzugsweise enthalten diese neben den erfindungsgemäßen Wirkstoffkombinationen zusätzlich mindestens eine UV-A-Filtersubstanz und/oder mindestens eine UV-B-Filtersubstanz und/oder mindestens ein anorganisches Pigment.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden z.B. in Tagescremes gewöhnlich UV-A- bzw. UV-B-Filtersubstanzen eingearbeitet.

Auch stellen UV-Schutzsubstanzen, ebenso wie Antioxidantien und, gewünschtenfalls, Konservierungsstoffe, einen wirksamen Schutz der Zubereitungen selbst gegen Verderb dar.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Enthalten die erfindungsgemäßen Emulsionen UVB-Filtersubstanzen, können diese öllöslich oder wasserlöslich sein. Erfindungsgemäß vorteilhafte öllösliche UVB-Filter sind z.B.:
- 3-Benzylidencampher-Derivate, vorzugsweise 3-(4-Methylbenzyliden)campher, 3-Benzylidencampher;
- 4-Aminobenzoësäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester,
- Derivate des 1,3,5-Triazins, vorzugsweise 2,4,6-Trianilino-(p-carbo-2'-ethyl-1'hexyloxy) -1,3,5-triazin.

Die Liste der genannten UVB-Filter, die in Kombination mit den erfindungsgemäßen Wirkstoffkombinationen verwendet werden können, soll selbstverständlich nicht limitierend sein.

Es kann auch von Vorteil sein, erfindungsgemäße Lipodispersionen mit UVA-Filtem zu formulieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion.

Erfindungsgemäße kosmetische und dermatologische Zubereitungen können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid.

Als weitere Bestandteile können verwendet werden:
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, - monoethyl- oder -monobutylether, Diethylenglykolmonomethyl- oder -monoethylether und analoge Produkte.

Erfindungsgemäße Zubereitungen können auch Wirkstoffe (eine oder mehrere Verbindungen) enthalten, welche gewählt werden aus der Gruppe: Acetylsalicylsäure, Atropin, Azulen, Hydrocortison und dessen Derivaten, z.B. Hydrocortison-17-valerat, Vitamine, z.B. Ascorbinsäure und deren Derivate, Vitamine der Bund D-Reihe, sehr günstig das Vitamin B1, das Vitamin B12 das Vitamin D1, aber auch Bisabolol, ungesättigte Fettsäuren, namentlich die essentiellen Fettsäuren (oft auch Vitamin F genannt), insbesondere die γ-Linolensäure, Ölsäure, Eicosapentaënsäure, Docosahexaensäure und deren Derivate, Chloramphenicol, Coffein, Prostaglandine, Thymol, Campher, Extrakte oder andere Produkte pflanzlicher und tierischer Herkunft, z.B. Nachtkerzenöl, Borretschöl oder Johannisbeerkemöl, Fischöle, Lebertran aber auch Ceramide und ceramidähnliche Verbindungen und so weiter. Vorteilhaft ist es auch, die Wirkstoffe aus der Gruppe der rückfettenden Substanzen zu wählen, beispielsweise Purcellinöl, Eucerit® und Neocerit®.

Die Menge solcher Wirkstoffe (eine oder mehrere Verbindungen) in den Zubereitungen gemäß der Erfindung beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

| Beispiel 1 (W/O-Creme): | |
|---|---|
| | Gew.-% |
| Cetyldimethiconcopolyol | 1,50 |
| Caprylsäure/Caprinsäuretriglyceride | 4,00 |
| Dicaprylylether | 3,00 |
| Octyldodecanol | 3,00 |
| Glycerin | 3,00 |
| Natriumchlorid | 0,70 |
| Chitosan | 0,25 |
| Milchsäure (90%ig) | 0,20 |
| Parfüm, Konservierungsmittel, Farbstoffe | q.s. |
| Wasser | ad 100,00 |

| Beispiel 2 (W/O-Creme): | |
|---|---|
| | Gew.-% |
| Laurylmethiconcopolyol | 2,00 |
| Octyldodecanol | 1,00 |
| C12-15 Alkylbenzoate | 1,00 |
| Squalan | 1,00 |
| Paraffinum liquidum | 6,00 |
| Glycerin | 3,00 |
| Natriumchlorid | 0,70 |
| Chitosan | 0,75 |
| Milchsäure (90%ig) | 0,60 |
| Parfüm, Konservierungsmittel, Farbstoffe | q.s. |
| Wasser | ad 100,00 |

| Beispiel 3 (W/O-Creme): | |
|---|---|
| | Gew.-% |
| Cetyldimethiconcopolyol | 2,00 |
| Squalan | 2,00 |
| Paraffinum liquidum | 3,00 |
| Stearylheptanoat | 1,00 |
| Hydrogeniertes Polysiobuten | 3,00 |
| Glycerin | 3,00 |
| Natriumchlorid | 0,70 |
| Chitosan | 1,25 |
| Milchsäure (90%ig) | 1,00 |
| Parfüm, Konservierungsmittel, Farbstoffe | q.s. |
| Wasser | ad 100,00 |

| Beispiel 4 (W/O-Creme): | |
|---|---|
| | Gew.-% |
| Laurylmethiconcopolyol | 2,00 |
| Paraffinum liquidum | 9,00 |
| Tocopherolacetat | 0,50 |
| Glycerin | 3,00 |
| Panthenol | 0,30 |
| 1,3-Butylenglycol | 1,00 |
| Serin | 0,30 |
| Biotin | 0,10 |
| Distärkephosphat | 1,00 |
| Natriumchlorid | 0,70 |
| Chitosan | 1,20 |
| Milchsäure (90%ig) | 1,00 |
| Parfüm, Konservierungsmittel, Farbstoffe | q.s. |
| Wasser | ad 100,00 |

| Beispiel 5 (W/O-Creme): | |
|---|---|
| | Gew.-% |
| Cetyldimethiconcopolyol | 2,00 |
| Isohexadecan | 2,00 |
| Paraffinum liquidum | 2,00 |
| Butylmethoxydibenzoylmethan | 1,00 |
| Octylmethoxycinnamat | 2,00 |
| Methylbenzylidencampher | 1,50 |
| Octyltriazon | 0,50 |
| Titandioxid | 1,00 |
| Zinkoxid | 1,00 |
| Glycerin | 1,00 |
| Natriumchlorid | 0,70 |
| Chitosan | 0,80 |
| Milchsäure (90%ig) | 0,70 |
| Parfüm, Konservierungsmittel, Farbstoffe | q.s. |
| Wasser | ad 100,00 |

| Beispiel 6 (W/O-Creme): | |
|---|---|
| | Gew.-% |
| Laurylmethiconcopolyol | 1,50 |
| Isohexadecan | 4,50 |
| Paraffinum subliquidum | 4,50 |
| Glycerin | 3,00 |
| Natriumchlorid | 0,70 |
| Chitosan | 0,40 |
| Milchsäure (90%ig) | 0,30 |
| Parfüm, Konservierungsmittel, Farbstoffe | q.s. |
| Wasser | ad 100,00 |

| Beispiel 7 (W/O-Creme): | |
|---|---|
| | Gew.-% |
| Cetyldimethiconcopolyol | 1,50 |
| Caprylsäure/Caprinsäuretriglyceride | 4,00 |
| Dicaprylylether | 2,50 |
| Octyldodecanol | 2,50 |
| Glycerin | 30,00 |
| Propylenglycol | 5,00 |
| Magnesiumsulfat | 0,70 |
| Polyquatemium-10 | 1,00 |
| Parfüm, Konservierungsmittel, Farbstoffe | q.s. |
| Wasser | ad 100,00 |

| Beispiel 8 (Wasser-in-Silicon-Creme): | |
|---|---|
| | Gew.-% |
| Laurylmethiconcopolyol | 1,50 |
| Cetyldimethiconcopolyol | 0,50 |
| Cyclomethicon | 9,00 |
| Sorbitol | 15,00 |
| Natriumchlorid | 0,70 |
| Chitosan | 1,30 |
| Milchsäure (90%ig) | 1,00 |
| Parfüm, Konservierungsmittel, Farbstoffe | q.s. |
| Wasser | ad 100,00 |

| Beispiel 9 (Wasser-in-Silicon-Creme): | |
|---|---|
| | Gew.-% |
| Cetyldimethiconcopolyol | 1,00 |
| Laurylmethiconcopolyol | 0,50 |
| Cyclomethicon | 8,00 |
| Dimethicon | 2,00 |
| Glycerin | 5,00 |
| Magnesiumsulfat | 0,70 |
| Polyquaternium-10 | 1,00 |
| Parfüm, Konservierungsmittel, Farbstoffe | q.s. |
| Wasser | ad 100,00 |

| Beispiel 10 (W/O-Creme): | |
|---|---|
| | Gew.-% |
| Laurylmethiconecopolyol | 1,00 |
| Cetyldimethiconcopolyol | 1,00 |
| Sonnenblumenöl | 3,00 |
| Dicaprylylether | 3,00 |
| Paraffinum liquidum | 4,00 |
| Glycerin | 3,00 |
| Natriumchlorid | 0,70 |
| Chitosan | 1,50 |
| Milchsäure (90%ig) | 1,20 |
| Parfüm, Konservierungsmittel, Farbstoffe | q.s. |
| Wasser | ad 100,00 |

| Beispiel 11 (W/O-Creme): | |
|---|---|
| | Gew.-% |
| Cetyldimethiconcopolyol | 1,50 |
| Caprylsäure/Caprinsäuretriglyceride | 4,00 |
| Jojobaöl | 2,00 |
| Dimethicon | 1,00 |
| Dimethiconol | 0,10 |
| Cyclomethicon | 2,00 |
| Dimethiconcopolyol | 0,20 |
| Distärkephosphat | 1,00 |
| Glycerin | 3,00 |
| Natriumchlorid | 0,70 |
| Chitosan | 0,80 |
| Milchsäure (90%ig) | 0,60 |
| Parfüm, Konservierungsmittel, Farbstoffe | q.s. |
| Wasser | ad 100,00 |

| Beispiel 12 (Emulsions-Make-up): | |
|---|---|
| | Gew.-% |
| Cetyldimethiconcopolyol | 1,50 |
| Octyldodecanol | 2,00 |
| C12-15-Alkylbenzoate | 2,00 |
| Squalan | 1,00 |
| Paraffinum liquidum | 1,00 |
| Distärkephosphat | 0,50 |
| Dimethicon | 0,50 |
| Glycerin | 1,50 |
| Chitosan | 0,80 |
| Milchsäure (90%ig) | 0,50 |
| Magnesiumsilikat | 1,00 |
| Glimmer | 0,50 |
| Eisenoxide | 0,50 |
| Titandioxid | 1,00 |
| Talkum | 1,00 |
| Tapioka Stärke | 0,25 |
| Parfüm, Konservierungsmittel, Farbstoffe | q.s. |
| Wasser | ad 100,00 |

### Allgemeine Anmerkungen zu den Beispielrezepturen:

1) pH-Wert der Wasserphase der Emulsion durch Zugabe von Säuren, die mit Chitosan in Wasser lösliche Salze bilden (wie z.B. Milchsäure, Glykolsäure, Essigsäure, Salicylsäure, Asparaginsäure) auf pHWasserphase < 6,0 (vorzugsweise 3,5 bis 5,5) einstellen.
2) Chitosan: mittleres Molekulargewicht von 50.000 bis 2.000.000 g/mol [bestimmt mittels Gelpermeationschromatographie] und einem Deacylierungsgrad von 10 bis 99 % [bestimmt mittels 1H-NMR].

## Patentansprüche

1. Wasser-in-Öl-Emulsionen mit
(a) einem Gehalt an Wasser und gegebenenfalls wasserlöslichen Substanzen von insgesamt mindestens 80 Gew.-%, bezogen auf das Gesamtgewicht der Zubereitungen und einem Gehalt an Lipiden, Emulgatoren und lipophilen Bestandteilen von insgesamt höchstens 20 Gew.-%.
(b) enthaltend wenigstens eine grenzflächenaktive Substanz, gewählt aus der Gruppe der Alkylmethiconcopolyole und/oder Alkyl-Dimethiconcopolyole,
(c) enthaltend eine oder mehrere Lipidkomponenten, welche die Lipidphase der Emulsion umfassen
(d) wobei das Gewichtsverhältnis von (b) zu (c) aus dem Bereich 0,10 bis 0,25 gewählt wird,
(e) femer enthaltend mindestens ein kationisches Polymer.

2. Emulsionen nach Anspruch 1, **dadurch gekennzeichnet, daß** als grenzflächenaktive Substanz Cetyl Dimethiconcopolyol gewählt wird.

3. Emulsionen nach Anspruch 1, **dadurch gekennzeichnet, daß** als grenzflächenaktive Substanz das Laurylmethiconcopolyol gewählt wird.

4. Emulsionen nach Anspruch 1, **dadurch gekennzeichnet, daß** die Gesamtmenge an den Alkylmethiconcopolyolen und/oder Alkyl-Dimethiconcopolyolen aus dem Bereich von 0,075 - 7,5 Gew.-%, bevorzugt 0,1- 5,0 Gew.-%, insbesondere 1,0 - 3,0 Gew.-% gewählt, bezogen auf das Gesamtgewicht der Zubereitungen.

5. Emulsionen nach Anspruch 1, **dadurch gekennzeichnet, daß** sie mehr als 85 Gew.-%, insbesondere mehr als 88 Gew.-% an Wasser und wasserlöslichen Substanzen enthalten, bezogen auf das Gesamtgewicht der Zubereitungen.

6. Emulsionen nach Anspruch 1, **dadurch gekennzeichnet, daß** sie 0,01 bis 10%, bevorzugt 0,25 - 1,25 % an kationischen Polymeren enthalten.

7. Emulsionen nach Anspruch 1, **dadurch gekennzeichnet, daß** das oder die kationischen Polymere gewählt werden aus der Gruppe der kationische Cellulosederivate, kationische Stärke, Copolymere von Diallylammoniumsalzen und Acrylamiden, quatemisierte Vinylpyrrolidon/Vinyl-imadazol-Polymere, Kondensationsprodukte von Polyglycolen und Aminen, quatemisierte Kollagenpolypeptide, quatemisierte Weizenpolypeptide, Polyethylenimin, kationische Silikonpolymere, Copolymere der Adipinsäure mit Dimethylaminohydroxypropyldietylentriamin, Copolymere der Acrylsäure mit Dimethyldiallylamoniumchlorid, Polyaminopolyamide, kationische Chitinderivate, kationischer Guar-Gum, quatemisierte Ammoniumsalz-Polymere sowie kationische Biopolymere wie z.B. Chitosan (mittleres Molekulargewicht von 50.000 bis 2.000.000 g/mol [bestimmt mittels Gelpermeationschromatographie] und einem Deacylierungsgrad von 10 bis 99% [bestimmt mittels 1H-NMR]).

## Claims

1. Water-in-oil emulsions
(a) with a content of water and optionally water-soluble substances totalling at least 80% by weight, based on the total weight of the preparations, and with a content of lipids, emulsifiers and lipophilic constituents totalling at most 20% by weight,
(b) comprising at least one surface-active substance chosen from the group of alkylmethicone copolyols and/or alkyldimethicone copolyols,
(c) comprising one or more lipid components which include the lipid phase of the emulsion,
(d) where the weight ratio of (b) to (c) is chosen from the range 0.10 to 0.25,
(e) and also comprising at least one cationic polymer.

2. Emulsions according to Claim 1, **characterized in that** the surface-active substance chosen is cetyldimethicone copolyol.

3. Emulsions according to Claim 1, **characterized in that** the surface-active substance chosen is laurylmethicone copolyol.

4. Emulsions according to Claim 1, **characterized in that** the total amount of alkylmethicone copolyols and/or alkyldimethicone copolyols is chosen from the range 0.075-7.5% by weight, preferably 0.1-5.0% by weight, in particular 1.0-3.0% by weight, based on the total weight of the preparations.

5. Emulsions according to Claim 1, **characterized in that** they comprise more than 85% by weight, in particular more than 88% by weight, of water and water-soluble substances, based on the total weight of the preparations.

6. Emulsions according to Claim 1, **characterized in that** they comprise from 0.01 to 10%, preferably 0.25-1.25%, of cationic polymers.

7. Emulsions according to Claim 1, **characterized in that** the cationic polymer(s) is/are chosen from the group consisting of cationic cellulose derivatives, cationic starch, copolymers of diallylammonium salts and acrylamides, quatemized vinypyrrolidone/vinylimidazole polymers, condensation products of polyglycols and amines, quaternized collagen polypeptides, quatemized wheat polypeptides, polyethyleneimine, cationic silicone polymers, copolymers of adipic acid with dimethylaminohydroxypropyldiethylenetriamine, copolymers of acrylic acid with dimethyldiallylammonium chloride, polyaminopolyamides, cationic chitin derivatives, cationic guar gum, quatemized ammonium salt polymers, and cationic biopolymers, such as, for example, chitosan (average molecular weight from 50,000 to 2,000,000 g/mol [determined by means of gel permeation chromatography] and a degree of deacylation of from 10 to 99% [determined by means of ¹H-NMR].

## Revendications

1. Emulsions d'eau dans l'huile, comprenant :
(a) une teneur globale en eau et éventuellement en substances solubles dans l'eau d'au moins 80% en poids par rapport au poids total des préparations, et une teneur globale en lipides, en émulsifiants et en composants lipophiles de maximum 20% en poids,
(b) au moins une substance tensioactive sélectionnée dans le groupe des copolyols d'alkylméthicone et/ou des copolyols d'alkyldiméthicone,
(c) un ou plusieurs composants lipidiques qui comprennent la phase lipidique de l'émulsion,
(d) le rapport pondéral entre (b) et (c) étant sélectionné dans la plage de 0,10 à 0,25,
(e) et comprenant en outre au moins un polymère cationique.

2. Emulsions selon la revendication 1, **caractérisées en ce que** l'on sélectionne comme substance tensioactive le copolyol de cétyldiméthicone.

3. Emulsions selon la revendication 1, **caractérisées en ce que** l'on sélectionne comme substance tensioactive le copolyol de laurylméthicone.

4. Emulsions selon la revendication 1, **caractérisées en ce que** la quantité totale de copolyols d'alkylméthicone et/ou de copolyols d'alkyldiméthicone est sélectionnée dans la plage de 0,075 à 7,5% en poids, de préférence de 0,1 à 5,0% en poids et en particulier de 1,0 à 3,0% en poids, par rapport au poids total des préparations.

5. Emulsions selon la revendication 1, **caractérisées en ce qu'**elles contiennent plus de 85% en poids, et en particulier plus de 88% en poids d'eau et de substances solubles dans l'eau par rapport au poids total des préparations.

6. Emulsions selon la revendication 1, **caractérisées en ce qu'**elles contiennent de 0,01 à 10%, de préférence de 0,25 à 1,25%, de polymères cationiques.

7. Emulsions selon la revendication 1, **caractérisées en ce que** le ou les polymères cationiques sont sélectionnés dans le groupe des dérivés cationiques de cellulose, des amidons cationiques, des copolymères de sels de diallylammonium et d'acrylamide, des polymères quaternisés de vinylpyrrolidone/vinylimidazol, des produits de condensation de polyglycols et d'amines, des polypeptides quatemisés de collagène, des polypeptides quatemisés de froment, des polyéthylèneimines, des polymères cationiques de silicone, des copolymères d'acide adipique et de diméthylaminohydroxypropyldiéthylènetriamine, des copolymères d'acide acrylique et de chlorure de diméthyldiallylammonium, des polyaminopolyamides, des dérivés cationiques de chitine, de la gomme de guar cationisée, des polymères quatemisés de sels d'ammonium ainsi que des biopolymères cationiques, par exemple le chitosan (poids moléculaire moyen de 50 000 à 2 000 000 g/mole [déterminé par chromatographie par perméation d'un gel] et avec un degré de désacylation de 10 à 99% [déterminé par RMN de ¹H]).
